# EUROPÄISCHE PATENTANMELDUNG

(11) **EP 1 216 694 A1**
(43) Veröffentlichungstag der Anmeldung: **26.06.2002**
(21) Anmeldenummer: 01129342.0
(22) Anmeldetag: 17.12.2001
(51) Int. Cl.: A61K 7/48, A61K 31/385

(54) **Wirkstoffkombinationen aus oberflächenaktiven Citronensäureestern und alpha-Liponsäure sowie kosmetische und dermatologische Zubereitungen mit einem Gehalt an solchen Gemischen**

(30) Priorität: 18.12.2000 DE 10063129
(71) Anmelder: Beiersdorf Aktiengesellschaft, 20245 Hamburg (DE)
(72) Erfinder: Raschke, Thomas, Dr., 22529 Pinneberg (DE); Kruse, Inge, 20146 Hamburg (DE); Schönruck, Uwe, Dr., 23866 Nahe (DE)

(57) **Zusammenfassung**

Kombinationen aus
(a) einem oder mehreren partiell neutralisierten Estem von Monoglyceriden und/oder Diglyceriden gesättigter Fettsäuren mit Citronensäure
(b) α-Liponsäure.

## Beschreibung

Die vorliegende Erfindung betrifft Wirkstoffkombinationen aus oberflächenaktiven Citronensäureestem und α-Liponsäure sowie kosmetische und dermatologische Zubereitungen mit einem Gehalt an solchen Gemischen sowie die Verwendung von oberflächenaktiven Citronensäureestern zur Stabilisierung von α-Liponsäure gegenüber chemischen Abbaureaktionen, insbesondere photochemischen Abbaureaktionen und/oder oxidationsbedingten Abbaureaktionen.

Darüberhinaus betrifft die Erfindung synergistische Gemische aus α-Liponsäure und oberflächenaktiven Substanzen sowie kosmetische und dermatologische Zubereitungen mit einem Gehalt an solchen Gemischen. Bevorzugt betrifft die vorliegende Erfindung kosmetische Zubereitungen mit einem wirksamen Schutz vor schädlichen Oxidationsprozessen in der Haut, aber auch zum Schutze kosmetischer Zubereitungen selbst bzw. zum Schutze der Bestandteile kosmetischer Zubereitungen vor schädlichen Oxidationsprozessen.

Die vorliegende Erfindung betrifft dementsprechend in bevorzugten Ausführungsformen kosmetische bzw. dermatologische dermatologische Zubereitungen, enthaltend Wirkstoffe zur Pflege und zum Schutze der Haut, insbesondere der empfindlichen Haut wie auch ganz besonders im Vordergrunde stehend der durch intrinsische und/oder extrinsische Faktoren gealterten oder alternden Haut sowie die Verwendung solcher Wirkstoffe und Kombinationen solcher Wirkstoffe auf dem Gebiete der kosmetischen und dermatologischen Hautpflege.

Die menschliche Haut übt als größtes Organ des Menschen zahlreiche lebenswichtige Funktionen aus. Mit durchschittlich etwa 2 m² Oberfläche beim Erwachsenen kommt ihr eine herausragende Rolle als Schutz- und Sinnesorgan zu. Aufgabe dieses Organs ist es, mechanische, thermische, aktinische, chemische und biologische Reize zu vermitteln und abzuwehren. Außerdem kommt ihr eine bedeutende Rolle als Regulations- und Zielorgan im menschlichen Stoffwechsel zu.

Unter kosmetischer Hautpflege ist in erster Linie zu verstehen, die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) zu stärken oder wiederherzustellen sowie ihre Hornschicht bei aufgetretenen Schäden in ihrem natürlichen Regenerationsvermögen zu unterstützen.

Werden die Barriereeigenschaften der Haut gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Die chronologische Hautalterung wird z.B. durch endogene, genetisch determinierte Faktoren verursacht. In Epidermis und Dermis kommt es alterungsbedingt z.B. zu folgenden Strukturschäden und Funktionsstörungen, die auch unter den Begriff "Senile Xerosis" fallen können:
a) Trockenheit, Rauhigkeit und Ausbildung von Trockenheitsfältchen,
b) Juckreiz und
c) verminderte Rückfettung durch Talgdrüsen (z.B. nach Waschen).
   Exogene Faktoren, wie UV-Licht und chemische Noxen, können kumulativ wirksam sein und z.B. die endogenen Alterungsprozesse beschleunigen bzw. sie ergänzen. In Epidermis und Dermis kommt es insbesondere durch exogene Faktoren z.B. zu folgenden Strukturschäden- und Funktionsstörungen in der Haut, die über Maß und Qualität der Schäden bei chronologischer Alterung hinausgehen:
d) Sichtbare Gefäßerweiterungen (Teleangiektasien, Cuperosis);
e) Schlaffheit und Ausbildung von Falten;
f) lokale Hyper-, Hypo- und Fehlpigmentierungen (z.B. Altersflecken) und
g) vergrößerte Anfälligkeit gegenüber mechanischem Stress (z.B. Rissigkeit).

Die vorliegende Erfindung betrifft insbesondere Produkte zur Pflege der auf natürliche Weise gealterten Haut, sowie zur Behandlung der Folgeschäden der Lichtalterung, insbesondere der unter a) bis g) aufgeführten Phänomene.

Produkte zur Pflege gealterter Haut sind an sich bekannt. Sie enthalten z.B. Retinoide (Vitamin A-Säure und/oder deren Derivate) bzw. Vitamin A und/oder dessen Derivate. Ihre Wirkung auf die Strukturschäden ist allerdings umfangsmäßig begrenzt. Darüber hinaus gibt es bei der Produktentwicklung erhebliche Schwierigkeiten, die Wirkstoffe in ausreichendem Maße gegen oxidativen Zerfall zu stabilisieren. Die Verwendung Vitamin A-Säure-haltiger Produkte bedingt darüber hinaus oft starke erythematöse Hautreizungen. Retinoide sind daher nur in geringen Konzentrationen einsetzbar.

Insbesondere betrifft die vorliegende Erfindung kosmetische Zubereitungen mit einem wirksamen Schutz vor schädlichen Oxidationsprozessen in der Haut, aber auch zum Schutze kosmetischer Zubereitungen selbst bzw. zum Schutze der Bestandteile kosmetischer Zubereitungen vor schädlichen Oxidationsprozessen.

Die vorliegende Erfindung betrifft ferner Antioxidantien, bevorzugt solche, welche in hautpflegenden kosmetischen oder dermatologischen Zubereitungen eingesetzt werden. Insbesondere betrifft die Erfindung auch kosmetische und dermatologische Zubereitungen, solche Antioxidantien enthaltend. In einer bevorzugten Ausführungsform betrifft die vorliegende Erfindung kosmetische und dermatologische Zubereitungen zur Prophylaxe und Behandlung kosmetischer oder dermatologischer Hautveränderungen wie z.B. der Hautalterung, insbesondere der durch oxidative Prozesse hervorgerufenen Hautalterung.

Weiterhin betrifft die vorliegende Erfindung Wirkstoffe und Zubereitungen, solche Wirkstoffe enthaltend, zur kosmetischen und dermatologischen Behandlung oder Prophylaxe erythematöser, entzündlicher, allergischer oder autoimmunreaktiver Erscheinungen, insbesondere Dermatosen.

Die vorliegende Erfindung betrifft in einer weiteren vorteilhaften Ausführungsform Wirkstoffkombinationen und Zubereitungen, die zur Prophylaxe und Behandlung der lichtempfindlichen Haut, insbesondere von Photodermatosen, dienen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, des sogenannten UVA-Bereich, ist es wichtig, Filtersubstanzen zur Verfügung zu haben, da dessen Strahlen Reaktionen bei lichtempfindlicher Haut hervorrufen können. Es ist erwiesen, daß UVA-Strahlung zu einer Schädigung der elastischen und kollagenen Fasern des Bindegewebes führt, was die Haut vorzeitig altem läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Zum Schutz gegen die Strahlen des UVA-Bereichs werden daher gewisse Derivate des Dibenzoylmethans verwendet, deren Photostabilität (Int. J. Cosm. Science 10, 53 (1988)), nicht in ausreichendem Maße gegeben ist.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, beispielsweise Hydroxyradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, daß auch ionische Spezies bei UV-Exposition entstehen, welche dann ihrerseits oxidativ in die biochemischen Prozesse einzugreifen vermögen.

Um diesen Reaktionen vorzubeugen, können den kosmetischen bzw. dermatologischen Formulierungen zusätzliche Antioxidantien und/oder Radikalfänger einverleibt werden.

Es ist bereits vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Aufgabe der Erfindung war es daher auch, kosmetische, dermatologische und pharmazeutische Wirkstoffe und Zubereitungen sowie Lichtschutzformulierungen zu schaffen, die zur Prophylaxe und Behandlung lichtempfindlicher Haut, insbesondere Photodermatosen, bevorzugt PLD dienen.

Weitere Bezeichnungen für die polymorphe Lichtdermatose sind PLD, PLE, Mallorca-Akne und eine Vielzahl von weiteren Bezeichnungen, wie sie in der Literatur (z.B. A. Voelckel et al, Zentralblatt Haut- und Geschlechtskrankheiten (1989), 156, S.2), angegeben sind.

Erythematöse Hauterscheinungen treten auch als Begleiterscheinungen bei gewissen Hauterkrankungen oder -unregelmäßigkeiten auf. Beispielsweise ist der typische Hautausschlag beim Erscheinungsbild der Akne regelmäßig mehr oder weniger stark gerötet.

Hauptsächlich werden Antioxidantien als Schutzsubstanzen gegen den Verderb der sie enthaltenden Zubereitungen verwendet. Dennoch ist bekannt, daß auch in der menschlichen und tierischen Haut unerwünschte Oxidationsprozesse auftreten können. Solche Prozesse spielen eine wesentliche Rolle bei der Hautalterung.

Im Aufsatz "Skin Diseases Associated with Oxidative Injury" in "Oxidative Stress in Dermatology", S. 323 ff. (Marcel Decker Inc., New York, Basel, Hong Kong, Herausgeber: Jürgen Fuchs, Frankfurt, und Lester Packer, Berkeley/Califomien), werden oxidative Schäden der Haut und ihre näheren Ursachen aufgeführt.

Auch aus dem Grunde, solchen Reaktionen vorzubeugen, können kosmetischen oder dermatologischen Formulierungen zusätzlich Antioxidantien und/oder Radikalfänger einverleibt werden.

Zwar sind einige Antioxidantien und Radikalfänger bekannt. So ist bereits in den US-Patentschriften 4,144,325 und 4,248,861 sowie aus zahlreichen anderen Dokumenten vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Übliche kosmetische Darreichungsformen sind Emulsionen. Darunter versteht man im allgemeinen ein heterogenes System aus zwei miteinander nicht oder nur begrenzt mischbaren Flüssigkeiten, die üblicherweise als Phasen bezeichnet werden. Die eine liegt dabei in Form von Tröpfchen vor (dispere oder innere Phase), während die andere Flüssigkeit eine kontinuierliche (kohärente oder innere Phase ) bildet. Seltenere Darreichungsformen sind multiple Emulsionen, also solche, welche in den Tröpfchen der dispergierten (oder diskontinuierlichen) Phase ihrerseits Tröpfchen einer weiteren dispergierten Phase enthalten, z.B. W/O/W-Emulsionen und O/W/O-Emulsionen.

Neuere Erkenntnisse führten in letzter Zeit zu einem besseren Verständnis praxisrelevanter kosmetischer Emulsionen. Dabei geht man davon aus, daß die im Überschuß eingesetzten Emulgatorgemische lamellare flüssigkristalline Phasen bzw. kristalline Gelphasen ausbilden. In der Gelnetzwerktheorie werden Stabilität und physikochemische Eigenschaften solcher Emulsionen auf die Ausbildung von viskoelastischen Gelnetzwerden zurückgeführt.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-ÖI-Emulsion (W/O-Emulsion, z. B. Butter) handelt es sich um das umgekehrte Prinzip, wobei der Grundcharakter hier durch das Öl bestimmt wird.

Um die Metastabilität von Emulsionen gewährleisten zu können, sind in der Regel grenzflächenaktive Substanzen, also Emulgatoren, nötig. An sich ist die Verwendung der üblichen kosmetischen Emulgatoren völlig unbedenklich. Dennoch können Emulgatoren, wie letztlich jede chemische Substanz, im Einzelfalle allergische oder auf Überempfindlichkeit des Anwenders beruhende Reaktionen hervorrufen. So ist bekannt, daß bei manchen besonders empfindlichen Personen bestimmte Lichtdermatosen durch gewisse Emulgatoren und gleichzeitige Einwirkung von Sonnenlicht ausgelöst werden.

Es ist möglich, emulgatorfreie Zubereitungen herzustellen, welche beispielsweise in einer wäßrigen Phase dispergierte Öltröpfchen, ähnlich einer O/W-Emulsion, aufweisen. Voraussetzung dafür kann sein, daß die kontinuierliche wäßrige Phase ein die dispergierte Phase stabilisierendes Gelgerüst aufweist und andere Umstände mehr. Solche Systeme werden gelegentlich Hydrodispersionen oder Oleodispersionen genannt, je nachdem, welches die disperse und welches die kontinuierliche Phase darstellt.

Es ist für die kosmetische Galenik aber weder nötig noch möglich, auf Emulgatoren ganz zu verzichten, zumal eine gewisse Auswahl an besonders milden Emulgatoren existiert. Allerdings besteht ein Mangel des Standes der Technik an einer befriedigend großen Vielfalt solcher Emulgatoren, welche dann auch das Anwendungsspektrum entsprechend milder und hautverträglicher kosmetischer Zubereitungen deutlich verbreitern würde.

So war eine Aufgabe der vorliegenden Erfindung, kosmetische bzw. dermatologische Zubereitungen mit hervorragenden hautpflegenden Eigenschaften zur Verfügung zu stellen.

α-Liponsäure wurde 1952 aus Lebergewebe isoliert und seine Struktur als schwefelhaltige Fettsäure aufgeklärt. Bakterien, Pflanzen und höhere Organismen können α-Liponsäure in ihrem Stoffwechsel selbst herstellen, für den Menschen ist die Frage einer eigenen Biosynthese noch offen.

α-Liponsäure wird zur Therapie der Polyneuropathie, einer Sensibilitätsstörung an Händen und Füßen als Spätfolge des Diabetes eingesetzt. 200 bis 600 Milligramm α-Liponsäure pro Tag führen zu einer signifikanten Verminderung der Schmerzintensität. Der Energiestoffwechsel der Hand- und Fußnerven wird durch α-Liponsäure aktiviert, dadurch kommt es zu einer besseren Nervenleitfähigkeit und damit zu weniger Taubheitsgefühlen und Reflexausfällen.

α-Liponsäure senkt pathologisch erhöhte Leberwerte und fördert die Ausheilung der Hepatitis. α-Liponsäure ist in den meisten Nahrungsmitteln in geringen Mengen enthalten, nur im Fleisch sind relativ hohe Werte zu finden. Es gilt als anerkannt, daß α-Liponsäure stark antioxidative Eigenschaften besitzt.

α-Liponsäure ist durch folgende chemische Struktur gekennzeichnet:

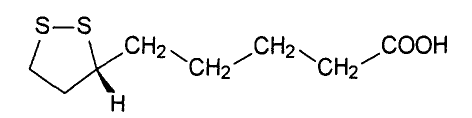

In der WO97/10808 und US-5,472,698 wird die kosmetische Verwendung der α-Liponsäure gegen Symptome der Hautalterung beschrieben. Die DE-42 42 876 beschreibt Wirkstoffkombinationen aus Biotin und Antioxidantien mit α-Liponsäure zur kosmetischen und/oder dermatologischen Pflege der Haut und/oder der Hautanhangsgebilde sowie kosmetische und/oder dermatologische Zubereitungen, solche Wirkstoffkombinationen enthaltend.

Es war indes überraschend und für den Fachmann nicht vorherzusehen, daß Kombinationen aus
(a) einem oder mehreren partiell neutralisierten Estem von Monoglyceriden und/oder Diglyceriden gesättigter Fettsäuren mit Citronensäure
(b) α-Liponsäure,
   zu gegenüber chemischen Abbaureaktionen, insbesondere photochemischen Abbaureaktionen und/oder oxidationsbedingten Abbaureaktionen stabilen Zubereitungen führt, die Bioverfügbarkeit der α-Liponsäure steigert, deren Wirksamkeit in synergistischer Weise erhöht und somit den Nachteilen des Standes der Technik abhilft.

Ein besonders vorteilhafter partiell neutralisierter Ester von Monoglyceriden und/oder Diglyceriden gesättigter Fettsäuren mit Citronensäure ist das Glycerylstearatcitrat. Solche Citronensäureester sind beispielsweise erhältlich unter der Produktbezeichnung "IMWI-TOR® 370" der Condea Chemie GmbH oder "Axol C 62" der Firma Goldschmidt AG.

Die Gesamtmenge an α-Liponsäure, wird vorteilhaft aus dem Bereich von 0,0001 - 10 Gew.-%, bevorzugt 0,005 - 5,0 Gew.-%, insbesondere 0,01 - 3,0 Gew.-% bezogen auf das Gesamtgewicht der Formulierung, gewählt.

Die Gesamtmenge an den erfindungsgemäß verwendeten partiell neutralisierten Estern von Monoglyceriden und/oder Diglyceriden gesättigter Fettsäuren mit Citronensäure in den fertigen kosmetischen oder dermatologischen Zubereitungen wird vorteilhaft aus dem Bereich von 0,1 - 20 Gew.-%, bevorzugt 0,5 - 10,0 Gew.-% insbesondere 1,0 - 5,0 Gew.-% gewählt, bezogen auf das Gesamtgewicht der Zubereitungen.

Es ist von Vorteil, Gewichtsverhältnisse zwischen α-Liponsäure einerseits und mindestens einem partiell neutralisierten Ester von Monoglyceriden und/oder Diglyceriden gesättigter Fettsäuren mit Citronensäure andererseits aus dem Bereich von 1 : 1 bis 1 : 500, bevorzugt 1 : 10 bis 1 zu 200, insbesondere 1 : 50 zu wählen.

Die erfindungsgemäße Kombination aus α-Liponsäure und mindestens einem partiell neutralisierten Ester von Monoglyceriden und/oder Diglyceriden gesättigter Fettsäuren mit Citronensäure wird im Rahmen dieser Schrift auch kollektiv als "erfindungsgemäßer Wirkstoff" oder "erfindungsgemäß verwendeteter Wirkstoff" oder "erfindungsgemäß verwendetete Wirkstoffkombination" bezeichnet bzw. mit sinnverwandten Bezeichnungen belegt.

Die erfindungsgemäßen Wirkstoffkombinationen lassen sich problemlos üblichen kosmetischen Zubereitungen, vorteilhaft Lichtschutzzubereitungen, aber auch gewünschtenfalls anderen Zubereitungen, beispielsweise pharmazeutischen Zubereitungen einverleiben.

Bei Anwendung des erfindungsgemäß verwendeten Wirkstoffes bzw. kosmetischer oder topischer dermatologischer Zubereitungen mit einem wirksamen Gehalt an erfindungsgemäß verwendetem Wirkstoff ist in überraschender Weise eine wirksame Behandlung, aber auch eine Prophylaxe
- von defizitären, sensitiven oder hypoaktiven Hautzuständen oder defizitären, sensitiven oder hypoaktiven Zustände von Hautanhangsgebilden
- von Erscheinungen vorzeitiger Alterung der Haut (z.B. Falten, Altersflecken, Teleangiektasien) und/oder der Hautanhangsgebilde,
- von umweltbedingten (Rauchen, Smog, reaktive Sauerstoffspecies, freie Radikale) und insbesondere lichtbedingten negativen Veränderungen der Haut und der Hautanhangsgebilde
- von trockener Haut
- von lichtbedingten Hautschäden
- von Pigmentierungsstörungen,
- von Juckreiz,
- von trockenen Hautzuständen und Hornschichtbarrierestörungen,
- von Haarausfall und für verbessertes Haarwachstum
- von entzündlichen Hautzuständen sowie atopischem Ekzem, seborrhoischem Ekzem, polymorpher Lichtdermatose, Psoriasis, Vitiligo möglich. Der erfindungsgemäße Wirkstoffes bzw. kosmetischer oder topischer dermatologischer Zubereitungen mit einem wirksamen Gehalt an erfindungsgemäßem Wirkstoff dient aber auch in überraschender Weise
- zur Beruhigung von empfindlicher oder gereizter Haut
- zur Stimulation der Kollagen-, Hyaluronsäure-, Elastinsynthese
- zur Stimulation der intrazellulären DNA-Synthese, insbesondere bei defizitären oder hypoaktiven Hautzuständen.
- zur Steigerung der Zellemeuerung und Regeneration der Haut
- zur Steigerung der hauteigenen Schutz- und Reparaturmechanismen (beispielsweise für dysfunktionelle Enzyme, DNA, Lipide, Proteine)
- zur Vor- und Nachbehandlung bei topischer Anwendung von Laser- und Abschleifbehandlungen, die z. B. der Reduzierung von Hautfalten und Narben dienen, um den resultierenden Hautreizungen entgegenzuwirken und die Regenerationsprozesse in der verletzten Haut zu fördern.

Es ist erfindungsgemäß insbesondere äußerst vorteilhaft, den erfindungsgemäß verwendeten Wirkstoff bzw. kosmetische oder topische dermatologische Zubereitungen mit einem wirksamen Gehalt an erfindungsgemäß verwendetem Wirkstoff zur kosmetischen oder dermatologischen Behandlung oder Prophylaxe unerwünschter Hautzustände zu verwenden.

Die Gesamtmenge an α-Liponsäure in den fertigen kosmetischen oder dermatologischen Zubereitungen wird vorteilhaft aus dem Bereich von 0,025 - 10,0 Gew.-%, bevorzugt 0,1 - 5,0 Gew.-% gewählt, bezogen auf das Gesamtgewicht der Zubereitungen.

Es war für den Fachmann daher nicht vorauszusehen gewesen, daß die erfindungsgemäß verwendeten Wirkstoffkombinationen bzw. kosmetische oder dermatologische Zubereitungen, solche enthaltend
- besser als Antioxidans wirken
- besser als Radikalfänger wirken
- besser die Bindung von schädlichen Photoprodukten an Lipide, DNS und Proteine verhindern
- besser gegen die Hautalterung wirken
- besser die Haut gegen Photoreaktionen schützen
- besser entzündlichen Reaktionen vorbeugen würde
   als die Wirkstoffe, Wirkstoffkombinationen und Zubereitungen des Standes der Technik. Ferner war nicht vorauszusehen gewesen, daß die erfindungsgemäß verwendeten Wirkstoffkombinationen in kosmetischen oder dermatologischen Zubereitungen höhere Stabilität aufweist als die jeweils einzeln verwendeten Wirkstoffe, was insbesondere α-Liponsäure betrifft.

Erfindungsgemäß sind daher die Verwendung von Wirkstoffkombinationen aus α-Liponsäure und mindestens einem partiell neutralisierten Ester von Monoglyceriden und/oder Diglyceriden gesättigter Fettsäuren mit Citronensäure als Antioxidans sowie seine Verwendung zur Bekämpfung und/oder Prophylaxe der durch oxidative Beanspruchung hervorgerufenen Hautalterung und entzündlicher Reaktionen.

Als besonders vorteilhafte Ausführungsform der vorliegenden Erfindung wird ferner angesehen die Verwendung von Wirkstoffkombinationen aus α-Liponsäure und mindestens einem partiell neutralisierten Ester von Monoglyceriden und/oder Diglyceriden gesättigter Fettsäuren mit Citronensäure zur Bekämpfung und/oder Prophylaxe des oxidativen Stresses.

Erfindungsgemäß können die kosmetischen oder dermatologischen Zubereitungen können wie üblich zusammengesetzt sein und zur Behandlung, der Pflege und der Reinigung der Haut und/oder der Haare und als Schminkprodukt in der dekorativen Kosmetik dienen. Sie enthalten bevorzugt 0,1 Gew.-% bis 20 Gew.-%, bevorzugt 0,5 Gew.-% bis 10 Gew.-%, insbesondere 1,0 - 5,0 Gew.-%, bezogen auf das Gesamtgewicht der Zubereitungen, an erfindungsgemäß verwendeten Wirkstoffkombinationen.

Es ist erfindungsgemäß bevorzugt, den erfindungsgemäß verwendeten Wirkstoffkombinationen bzw. kosmetischen oder dermatologischen Zubereitungen, solche Wirkstoffkombinationen enthaltend Komplexbildner zuzufügen.

Komplexbildner sind an sich bekannte Hilfsstoffe der Kosmetologie bzw. der medizinischen Galenik. Durch die Komplexierung von störenden Metallen wie Mn, Fe, Cu und anderer können beispielsweise unerwünschte chemische Reaktionen in kosmetischen oder dermatologischen Zubereitungen verhindert werden.

Komplexbildner, insbesondere Chelatoren, bilden mit Metallatomen Komplexe, welche bei Vorliegen eines oder mehrerer mehrbasiger Komplexbildner, also Chelatoren, Metallacyclen darstellen. Chelate stellen Verbindungen dar, in denen ein einzelner Ligand mehr als eine Koordinationsstelle an einem Zentralatom besetzt. In diesem Falle werden also normalerweise gestreckte Verbindungen durch Komplexbildung über ein Metall-Atom od. -Ion zu Ringen geschlossen. Die Zahl der gebundenen Liganden hängt von der Koordinationszahl des zentralen Metalls ab. Voraussetzung für die Chelatbildung ist, daß die mit dem Metall reagierende Verbindung zwei oder mehr Atomgruppierungen enthält, die als Elektronendonatoren wirken.

Der oder die Komplexbildner können vorteilhaft aus der Gruppe der üblichen Verbindungen gewählt werden, wobei bevorzugt mindestens eine Substanz aus der Gruppe bestehend aus Weinsäure und deren Anionen, Citronensäure und deren Anionen, Aminopolycarbonsäuren und deren Anionen (wie beispielsweise Ethylendiamintetraessigsäure (EDTA) und deren Anionen, Nitrilotriessigsäure (NTA) und deren Anionen, Hydroxyethylendiaminotriessigsäure (HOEDTA) und deren Anionen, Diethylenaminopentaessigsäure (DPTA) und deren Anionen, trans-1,2-Diaminocyclohexantetraessigsäure (CDTA) und deren Anionen).

Der oder die Komplexbildner sind erfindungsgemäß vorteilhaft in kosmetischen oder dermatologischen Zubereitungen bevorzugt zu 0,001 Gew.-% bis 10 Gew.-%, bevorzugt zu 0,01 Gew.-% bis 5 Gew.-%, insbesondere bevorzugt zu 0,05 - 2,0 Gew.-%, bezogen auf das Gesamtgewicht der Zubereitungen, enthalten.

Zur Anwendung werden die kosmetischen und dermatologischen Zubereitungen erfindungsgemäß in der für Kosmetika üblichen Weise auf die Haut und/oder die Haare in ausreichender Menge aufgebracht.

Erfindungsgemäß können kosmetische und dermatologische Zubereitungen in verschiedenen Formen vorliegen. Es ist insbesondere vorteilhaft, wenn sie eine Emulsion oder Mikroemulsion vom Typ Öl-in-Wasser (O/W), darstellen.

Es ist auch möglich und vorteilhaft im Sinne der vorliegenden Erfindung, erfindungsgemäß verwendete Wirkstoffkombinationen in wäßrige Systeme bzw. Tensidzubereitungen zur Reinigung der Haut und der Haare einzufügen.

Erfindungsgemäß können die kosmetischen und dermatologischen Zubereitungen kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Insbesondere können erfindungsgemäß verwendete Wirkstoffkombinationen auch mit anderen Antioxidantien und/oder Radikalfängern kombiniert werden.

Vorteilhaft werden solche Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α -Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Pyridoxin und dessen Derivate, Ubichinon und Ubichinol und deren Derivate, Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, Ascorbinsäure und deren Derivate, insbesondere Ascorbylpalmitat, Ascorbylphosphat und verwandte Verbindungen, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Sesamol, Sesamolin, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,025 - 20 Gew.-%, insbesondere 0,05 - 10 Gew.-%, bezogen auf das Gesamtgewicht der Zubereitung.

Sofern Ascorbinsäure und/oder deren Derivate das oder die zusätzlichen Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin E und/oder dessen Derivate das oder die zusätzlichen Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Erfindungsgemäß sind Emulsionen vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der Zubereitungen gemäß der Erfindung enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, beziehungsweise aus der Gruppe der sogenannten Pemulene, beispielsweise Pemulene der Typen TR-1 , TR-2, jeweils einzeln oder in Kombination.

Insbesondere werden Gemische der vorstehend genannten Lösemittel verwendet. Bei alkoholischen Lösemitteln kann Wasser ein weiterer Bestandteil sein.

Emulsionen gemäß der Erfindung enthalten vorteilhaft z.B. die genannten Fette, Öle, Wachse und anderen Fettkörper, sowie Wasser und gegebenenfalls einen oder mehrere weitere Emulgatoren, wie sie üblicherweise verwendet werden.

Als Emulsionen vorliegende Zubereitungen gemäß der Erfindung enthalten gegebenenfalls besonders vorteilhaft einen oder mehrere zusätzliche O/W-Emulgatoren. Solche O/W-Emulgatoren können beispielsweise vorteilhaft gewählt werden aus der Gruppe der polyethoxylierten bzw. polypropoxylierten bzw. polyethoxylierten und polypropoxylierten Produkte, z.B.:
- der Fettalkoholethoxylate
- der ethoxylierten Wollwachsalkohole,
- der Polyethylenglycolether der allgemeinen Formel

   R-O-(-CH₂-CH₂-O-)ₙ-R',
- der Fettsäureethoxylate der allgemeinen Formel

   R-COO-(-CH₂-CH₂-O-)ₙ-H,
- der veretherten Fettsäureethoxylate der allgemeinen Formel

   R-COO-(-CH₂-CH₂-O-)ₙ-R',
- der veresterten Fettsäureethoxylate der allgemeinen Formel

   R-COO-(-CH₂-CH₂-O-)ₙ-C(O)-R',
- der Polyethylenglycolglycerinfettsäureester
- der ethoxylierten Sorbitanester
- der Cholesterinethoxylate
- der ethoxylierten Triglyceride
- der Alkylethercarbonsäuren der allgemeinen Formel

   R-O-(-CH₂-CH₂-O-)ₙ-CH₂-COOH

   nd n eine Zahl von 5 bis 30 darstellen,
- der Polyoxyethylensorbitolfettsäureester,
- der Alkylethersulfate der allgemeinen Formel

   R-O-(-CH₂-CH₂-O-)ₙ-SO₃-H
- der Fettalkoholpropoxylate der allgemeinen Formel

   R-O-(-CH₂-CH(CH₃)-O-)ₙ-H,
- der Polypropylenglycolether der allgemeinen Formel

   R-O-(-CH₂-CH(CH₃)-O-)ₙ-R',
- der propoxylierten Wollwachsalkohole,
- der veretherten Fettsäurepropoxylate

   R-COO-(-CH₂-CH(CH₃)-O-)ₙ-R',
- der veresterten Fettsäurepropoxylate der allgemeinen Formel

   R-COO-(-CH₂-CH(CH₃)-O-)ₙ-C(O)-R',
- der Fettsäurepropoxylate der allgemeinen Formel

   R-COO-(-CH₂-CH(CH₃)-O-)ₙ-H,
- der Polypropylenglycolglycerinfettsäureester
- der propoxylierten Sorbitanester
- der Cholesterinpropoxylate
- der propoxylierten Triglyceride
- der Alkylethercarbonsäuren der allgemeinen Formel

   R-O-(-CH₂-CH(CH₃)O-)ₙ-CH₂-COOH
- der Alkylethersulfate bzw. die diesen Sulfaten zugrundeliegenden Säuren der allgemeinen Formel

   R-O-(-CH₂-CH(CH₃)-O-)ₙ-SO₃-H
- der Fettalkoholethoxylate/propoxylate der allgemeinen Formel

   R-O-Xₙ-Yₘ-H,
- der Polypropylenglycolether der allgemeinen Formel

   R-O-Xₙ-Yₘ-R',
- der veretherten Fettsäurepropoxylate der allgemeinen Formel

   R-COO-Xₙ-Yₘ-R',
- der Fettsäureethoxylate/propoxylate der allgemeinen Formel

   R-COO-Xₙ-Yₘ-H,.

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11 - 18, ganz besonders vorteilhaft mit mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:
Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)stearylether (Steareth-17), Polyethylenglycol-(18)stearylether (Steareth-18), Polyethylenglycol(19)stearylether (Steareth-19), Polyethylenglycol(20)stearylether (Steareth-20),
Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)isostearylether (Isosteareth-16), Polyethylenglycol(17)isostearylether (Isosteareth-17), Polyethylenglycol-(18)isostearylether (Isosteareth-18), Polyethylenglycol(19)isostearylether (Isosteareth-19), Polyethylenglycol(20)isostearylether (Isosteareth-20),
Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20)cetylether (Ceteth-20),
Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)-isocetylether (Isoceteth-16), Polyethylenglycol(17)isocetylether (Isoceteth-17), Polyethylenglycol(18)isocetylether (Isoceteth-18), Polyethylenglycol(19)isocetylether (Isoceteth-19), Polyethylenglycol(20)isocetylether (Isoceteth-20),
Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15),
Polyethylenglycol(12)laurylether (Laureth-12), Polyethylenglycol(12)isolaurylether (Isolaureth-12).
Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol-(19)cetylstearylether (Ceteareth-19), Polyethylenglycol(20)cetylstearylether (Ceteareth-20),

Es ist ferner von Vorteil, die Fettsäureethoxylate aus folgender Gruppe zu wählen:
Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat,
Polyethylenglycol(12)isostearat, Polyethylenglycol(13)isostearat, Polyethylenglycol-(14)isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21)isostearat, Polyethylenglycol-(22)isostearat, Polyethylenglycol(23)isostearat, Polyethylenglycol(24)isostearat, Polyethylenglycol(25)isostearat,
Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat

Als ethoxylierte Alkylethercarbonsäure bzw. deren Salz kann vorteilhaft das Natriumlaureth-1 1-carboxylat verwendet werden.

Als Alkylethersulfat kann Natrium Laureth 1-4 sulfat vorteilhaft verwendet werden.

Als ethoxyliertes Cholesterinderivat kann vorteilhaft Polyethylenglycol(30)Cholesterylether verwendet werden. Auch Polyethylenglycol(25)Sojasterol hat sich bewährt.

Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/caprinat, Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)-sorbitanmonooleat zu wählen.

Als Emulsionen vorliegende Zubereitungen gemäß der Erfindung enthalten aber auch gegebenenfalls vorteilhaft einen oder mehrere zusätzliche W/O-Emulgatoren. Als solche vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 - 18 C-Atomen, Diglycerinester gesättigter und/oder ungesättigter, verzweigter und/oder unverzweigter Alkancarbonsäuren einer Kettenlänge von 8 bis 24, insbesondere 12 - 18 C-Atomen, Monoglycerinether gesättigter und/oder ungesättigter, verzweigter und/oder unverzweigter Alkohole einer Kettenlänge von 8 bis 24, insbesondere 12 - 18 C-Atomen, Diglycerinether gesättigter und/oder ungesättigter, verzweigter und/oder unverzweigter Alkohole einer Kettenlänge von 8 bis 24, insbesondere 12 - 18 C-Atomen, Propylenglycolester gesättigter und/oder ungesättigter, verzweigter und/oder unverzweigter Alkancarbonsäuren einer Kettenlänge von 8 bis 24, insbesondere 12 - 18 C-Atomen sowie Sorbitanester gesättigter und/oder ungesättigter, verzweigter und/oder unverzweigter Alkancarbonsäuren einer Kettenlänge von 8 bis 24, insbesondere 12 - 18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Vorteilhaft können Zubereitungen gemäß der Erfindung außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Enthalten die Zubereitungen gemäß der Erfindung UVB-Filtersubstanzen, können diese öllöslich oder wasserlöslich sein. Erfindungsgemäß vorteilhafte öllösliche UVB-Filter sind z.B.:
- 3-Benzylidencampher-Derivate, vorzugsweise 3-(4-Methylbenzyliden)campher, 3-Benzylidencampher;
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester,
- 2,4,6-Trianilino-(p-carbo-2'-ethyl-1'-hexyloxy) -1,3,5-triazin.

Vorteilhafte wasserlösliche UVB-Filter sind z.B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bomylidenmethyl)benzolsulfonsäure, 2-Methyl-5-(2-oxo-3-bornylidenmethyl)sulfonsäure und ihre Salze sowie das 1,4-di(2-oxo-10-Sulfo-3-bomylidenmethyl)-Benzol und dessen Salze (die entprehenden 10-Sulfato-verbindungen, beispielsweise das entsprechende Natrium-, Kalium- oder Triethanolammonium-Salz), auch als Benzol-1,4-di(2-oxo-3-bornylidenmethyl-10-Sulfonsäure bezeichnet

Die Liste der genannten UVB-Filter, die in Kombination mit den erfindungsgemäßen Wirkstoffkombinationen verwendet werden können, soll selbstverständlich nicht limitierend sein.

Gegenstand der Erfindung ist auch die Verwendung einer Kombination der erfindungsgemäß verwendeten Wirkstoffkombinationen mit mindestens einem UVB-Filter als Antioxidans bzw. die Verwendung einer Kombination der erfindungsgemäß verwendeten Wirkstoffkombinationen mit mindestens einem UVB-Filter als Antioxidans in einer kosmetischen oder dermatologischen Zubereitung.

Es kann auch von Vorteil sein, die erfindungsgemäß verwendeten Wirkstoffkombinationen mit UVA-Filtern zu kombinieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Gegenstand der Erfindung ist auch die Verwendung einer Kombination von erfindungsgemäß verwendeten Wirkstoffkombinationen mit mindestens einem UVA-Filter als Antioxidans bzw. die Verwendung einer Kombination der erfindungsgemäßen Wirkstoffkombinationen mit mindestens einem UVA-Filter als Antioxidans in einer kosmetischen oder dermatologischen Zubereitung.

Gegenstand der Erfindung ist auch die Verwendung einer Kombination aus erfindungsgemäß verwendeten Wirkstoffkombinationen mit mindestens einem UVA-Filter und mindestens einem UVB-Filter als Antioxidans bzw. die Verwendung einer Kombination aus erfindungsgemäßen Wirkstoffen mit mindestens einem UVA-Filter und mindestens einem UVB-Filter als Antioxidans in einer kosmetischen oder dermatologischen Zubereitung.

Kosmetische und dermatologische Zubereitungen mit einem wirksamen Gehalt an erfindungsgemäß verwendeten Wirkstoffkombinationen können auch anorganische Pigmente enthalten, die üblicherweise in der Kosmetik zum Schutze der Haut vor UV-Strahlen verwendet werden. Dabei handelt es sich um Oxide des Titans, Zinks, Zirkoniums, Siliciums, Mangans, Cers und Mischungen davon, sowie Abwandlungen, bei denen die Oxide die aktiven Agentien sind. Besonders bevorzugt handelt es sich um Pigmente auf der Basis von Titandioxid.

Auch diese Kombinationen von UVA-Filter und Pigment bzw. Zubereitungen, die diese Kombination enthalten, sind Gegenstand der Erfindung. Es können die für die vorstehenden Kombinationen genannten Mengen verwendet werden.

Bei kosmetischen und dermatologischen Zubereitungen zum Schutze der Haare vor UV-Strahlen gemäß der Erfindung handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel.

Die kosmetischen und dermatologischen enthalten Wirkstoffe und Hilfsstoffe, wie sie üblicherweise für diesen Typ von Zubereitungen zur Haarpflege und Haarbehandlung verwendet werden. Als Hilfsstoffe dienen Konservierungsmittel, oberflächenaktive Substanzen, Substanzen zum Verhindern des Schäumens, Verdickungsmittel, Emulgatoren, Fette, Öle, Wachse, organische Lösungsmittel, Bakterizide, Parfüme, Farbstoffe oder Pigmente, deren Aufgabe es ist, die Haare oder die kosmetische oder dermatologische Zubereitung selbst zu färben, Elektroyte, Substanzen gegen das Fetten der Haare.

Unter Elektrolyten im Sinne der vorliegenden Erfindung sind wasserlösliche Alkali-, Ammonium-, Erdalkali- (unter Einbeziehung des Magnesiums) und Zinksalze anorganischer Anionen und beliebige Gemische aus solchen Salzen zu verstehen, wobei gewährleistet sein muß, daß sich diese Salze durch pharmazeutische oder kosmetische Unbedenklichkeit auszeichnen.

Die Anionen werden gemäß der Erfindung bevorzugt gewählt aus der Gruppe der Chloride, der Sulfate und Hydrogensulfate, der Phosphate, Hydrogenphosphate und der linearen und cyclischen Oligophosphate sowie der Carbonate und Hydrogencarbonate.

Kosmetische Zubereitungen, die ein Hautreinigungsmittel oder Shampoonierungsmittel darstellen, enthalten vorzugsweise mindestens eine anionische, nicht-ionische oder amphotere oberflächenaktive Substanz, oder auch Gemische aus solchen Substanzen, die erfindungsgemäß verwendeten Wirkstoffkombinationen im wäßrigen Medium und Hilfsmittel, wie sie üblicherweise dafür verwendet werden. Die oberflächenaktive Substanz bzw. die Gemische aus diesen Substanzen können in einer Konzentration zwischen 1 Gew.-% und 50 Gew.-% in dem Shampoonierungsmittel vorliegen.

Liegen die kosmetischen oder dermatologischen Zubereitungen in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wird, so handelt es sich dabei z.B. um wäßrige oder wäßrig-alkoholische Lösungen, die gegebenenfalls oberflächenaktive Substanzen enthalten, deren Konzentration zwischen 0,1 und 10 Gew.-%, vorzugsweise zwischen 0,2 und 5 Gew.-%, liegen kann.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält mindestens ein kationisches, anionisches, nicht-ionisches oder amphoteres Polymer oder auch Gemische derselben, sowie erfindungsgemäß verwendete Wirkstoffkombinationen in wirksamer Konzentration. Die Menge der verwendeten Polymeren liegt z.B. zwischen 0,1 und 10 Gew.-%, bevorzugt zwischen 0,1 und 3 Gew.-%.

Erfindungsgemäß können kosmetische Zubereitungen zur Behandlung und Pflege der Haare als Gele vorliegen, die neben einem wirksamen Gehalt an erfindungsgemäßen Wirkstoffen und dafür üblicherweise verwendeten Lösungsmitteln, bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z.B. Aluminiumsilikate wie beispielsweise Bentonite, oder ein Gemisch aus Polyethylenglykol und Polyethylenglycolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Vorzugsweise beträgt die Menge an erfindungsgemäßem Wirkstoff in einem für die Haare bestimmten Mittel 0,1 Gew.-% bis 10 Gew.-%, insbesondere 0,5 Gew.-% bis 5 Gew.-%, bezogen auf das Gesamtgewicht des Mittels.

Wäßrige kosmetische Reinigungsmittel oder für die wäßrige Reinigung bestimmte wasserarme oder wasserfreie Reinigungsmittelkonzentrate gemäß der Erfindung können anionische, nichtionische und/oder amphotere Tenside enthalten, beispielsweise
- herkömmliche Seifen, z.B. Fettsäuresalze des Natriums
- Alkylsulfate, Alkylethersulfate, Alkan- und Alkylbenzolsulfonate
- Sulfoacetate
- Sulfobetaine
- Sarcosinate
- Amidosulfobetaine
- Sulfosuccinate
- Sulfobernsteinsäurehalbester
- Alkylethercarboxylate
- Eiweiß-Fettsäure-Kondensate
- Alkylbetaine und Amidobetaine
- Fettsäurealkanolamide
- Polyglycolether-Derivate

Kosmetische Zubereitungen, die kosmetische Reinigungszubereitungen für die Haut darstellen, können in flüssiger oder fester Form vorliegen. Sie enthalten neben erfindungsgemäß verwendeten Wirkstoffkombinationen vorzugsweise mindestens eine anionische, nicht-ionische oder amphotere oberflächenaktive Substanz oder Gemische daraus, gewünschtenfalls einen oder mehrere Elektrolyten und Hilfsmittel, wie sie üblicherweise dafür verwendet werden. Die oberflächenaktive Substanz kann in einer Konzentration zwischen 1 und 94 Gew.-% in den Reinigungszubereitungen vorliegen, bezogen auf das Gesamtgewicht der Zubereitungen.

Kosmetische Zubereitungen, die ein Shampoonierungsmittel darstellen, enthalten neben einem wirksamen Gehalt an erfindungsgemäßem Wirkstoff vorzugsweise mindestens eine anionische, nicht-ionische oder amphotere oberflächenaktive Substanz oder Gemische daraus, gegebenenfalls einen erfindungsgemäßes Elektrolyten und Hilfsmittel, wie sie üblicherweise dafür verwendet werden. Die oberflächenaktive Substanz kann in einer Konzentration zwischen 1 Gew.-% und 94 Gew.-% in dem Shampoonierungsmittel vorliegen.

Die Zusammensetzungen gemäß der Erfindung enthalten außer gegebenenfalls den vorgenannten Tensiden Wasser und gegebenenfalls die in der Kosmetik üblichen Zusatzstoffe, beispielsweise Parfüm, Verdicker, Farbstoffe, Desodorantien, antimikrobielle Stoffe, rückfettende Agentien, Komplexierungs- und Sequestrierungsagentien, Periglanzagentien, Pflanzenextrakte, Vitamine, Wirkstoffe und dergleichen.

Die vorliegende Erfindung umfaßt auch ein kosmetisches Verfahren zum Schutze der Haut und der Haare vor oxidativen bzw. photooxidativen Prozessen, das dadurch gekennzeichnet ist, daß man ein kosmetisches Mittel, welches eine wirksame Konzentration an erfindungsgemäß verwendeten Wirkstoffkombinationen enthält, in ausreichender Menge auf die Haut oder Haare aufbringt.

Vorzugsweise beträgt die Menge an erfindungsgemäß verwendeten Wirkstoffkombinationen in diesen Zubereitungen 0,1 - 20 Gew.-%, bevorzugt 0,5 - 10 Gew.-%, insbesondere 1,0 - 5,0 Gew.-%, bezogen auf das Gesamtgewicht der Zubereitungen.

Gegenstand der Erfindung ist auch das Verfahren zur Herstellung der erfindungsgemäßen kosmetischen Mittel, das dadurch gekennzeichnet ist, daß man in an sich bekannter Weise erfindungsgemäßen Wirkstoffkombinationen in kosmetische und dermatologische Formulierungen einarbeitet.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **Beispiel 1 (O/W-Creme):** | |
|---|---|
| | Gew.-% |
| Glycerylstearatcitrat | 2,00 |
| Stearylalkohol | 4,00 |
| Caprylsäure/Caprinsäuretriglyceride | 3,00 |
| Octyldodecanol | 5,00 |
| Hydrierte Kokosfettsäureglyceride | 2,00 |
| Glycerin | 3,00 |
| Carbomer | 0,10 |
| α-Liponsäure | 0,20 |
| Natriumhydroxid | q.s. |
| Konservierung | q.s. |
| Parfum | q.s. |
| Wasser,demineralisiert | ad 100.0 |
| pH-Wert eingestellt auf 5.5 | |

| **Beispiel 2 (O/W-Lotion):** | |
|---|---|
| | Gew.-% |
| Glycerylstearatcitrat | 3,00 |
| Stearylalkohol | 2,00 |
| Caprylsäure/Caprinsäuretriglyceride | 1,00 |
| Dicaprylylether | 2,00 |
| Octyldodecanol | 1,00 |
| Glycerin | 3,00 |
| EDTA | 0,20 |
| Carbomer | 0,15 |
| α-Liponsäure | 0,10 |
| Natriumhydroxid | q.s. |
| Konservierung | q.s. |
| Parfum | q.s. |
| Wasser,demineralisiert | ad 100.0 |
| pH-Wert eingestellt auf 5.0 | |

| **Beispiel 3 (O/W-Creme):** | |
|---|---|
| | Gew.-% |
| Glycerylstearatcitrat | 3,00 |
| Cetylstearylalkohol | 4,00 |
| Paraffinöl | 3,00 |
| Caprylsäure-/Caprinsäuretriglyceride | 5,00 |
| Dicaprylylether | 5,00 |
| Xanthangummi | 0,10 |
| Citronensäure | 0,10 |
| Natriumcitrat | 0,20 |
| α-Liponsäure | 0,50 |
| Glycerin | 3,00 |
| Parfüm, Konservierungsmittel, Farbstoffe usw. | q.s. |
| Wasser | ad 100,00 |
| pH-Wert eingestellt auf 6.0 | |

| **Beispiel 4 (Emulsions-Make-up):** | |
|---|---|
| | Gew.-% |
| Glycerylstearatcitrat | 3,00 |
| Stearylalkohol | 1,00 |
| Caprylsäure-/Caprinsäuretriglyceride | 3,00 |
| Dimethicon | 1,00 |
| Glycerin | 3,00 |
| Carbomer | 0,15 |
| Glimmer | 1,00 |
| Magnesiumsilikat | 1,00 |
| Eisenoxide | 1,00 |
| Titandioxid | 2,50 |
| Talkum | 5,00 |
| α-Liponsäure | 0,30 |
| Natriumhydroxid | q.s. |
| Konservierung | q.s. |
| Parfum | q.s. |
| Wasser,demineralisiert | ad 100,00 |
| pH-Wert eingestellt auf 5.5 | |

| **Beispiel 5 (O/W-Creme):** | |
|---|---|
| | Gew.-% |
| Glycerylstearatcitrat | 3,00 |
| Cetylstearylalkohol | 3,00 |
| Octyldodecanol | 3,00 |
| Caprylsäure-/Caprinsäuretriglyceride | 5,00 |
| Squalan | 1,00 |
| Jojobaöl | 1,00 |
| Cyclomethicon | 3,00 |
| Dimethicon | 0,50 |
| Vitamin-E-Acetat | 1,00 |
| Paraffinum liquidum | 1,00 |
| Xanthangummi | 0,10 |
| α-Liponsäure | 1,00 |
| Retinylpalmitat | 0,20 |
| Glycerin | 3,00 |
| BHT Parfüm, Konservierungsmittel, | 0,10 |
| Farbstoffe | q.s. |
| Wasser | ad 100,00 |
| pH-Wert eingestellt auf 6.5 | |

| **Beispiel 6 (O/W-Lotion):** | |
|---|---|
| | Gew.-% |
| Glycerylstearatcitrat | 2,00 |
| Stearylalkohol | 1,00 |
| Octyldodecanol | 1,00 |
| Caprylsäure-/Caprinsäuretriglycerid | 1,00 |
| Tocopherylacetat | 2,00 |
| Natriumcarbomer | 0,10 |
| α-Liponsäure | 0,30 |
| Glycerin Parfüm, Konservierungsmittel, | 3,00 |
| Farbstoffe usw, | q.s. |
| Wasser | ad 100,00 |
| pH-Wert eingestellt auf 5,5 | |

| **Beispiel 7 (Sonnenschutz-Creme):** | |
|---|---|
| | Gew.-% |
| Glycerylstearatcitrat | 3,00 |
| Cetylstearylalkohol | 1,00 |
| Octyldodecanol | 3,00 |
| Paraffinöl | 3,00 |
| Dicaprylylether | 3,00 |
| Xanthangummi | 0,10 |
| Natriumcarbomer | 0,10 |
| Glycerin | 3,00 |
| α-Liponsäure | 0,15 |
| Ethylhexylmethoxycinnamat | 4,00 |
| Ethylhexylsalicylat | 2,00 |
| Dinatriumphenyldibenzimidazoltetrasulfonat | 1,00 |
| Titandioxid | 1,00 |
| Disodium EDTA | 0,10 |
| Parfüm, Konservierungsmittel, Farbstoffe, usw. | q.s. |
| Wasser | ad 100,00 |
| pH-Wert eingestellt auf 6.0 | |

| **Beispiel 8 (Sonnenschutz-Lotion):** | |
|---|---|
| | Gew.-% |
| Glycerylstearatcitrat | 3,00 |
| Stearylalkohol | 2,00 |
| Caprylsäure/Caprinsäuretriglyceride | 4,00 |
| Dicaprylylether | 2,00 |
| Octyldodecanol | 3,00 |
| Glycerin | 3,00 |
| Butylmethoxydibenzoylmethan | 1,00 |
| 4-Methylbenzylidencampher | 2,00 |
| Ethylhexyltriazon | 2,00 |
| EDTA | 0,20 |
| Carbomer | 0,15 |
| α-Liponsäure | 0,10 |
| Natriumhydroxid | q.s. |
| Konservierung | q.s. |
| Parfum | q.s. |
| Wasser,demineralisiert | ad 100,00 |
| pH-Wert eingestellt auf 5.0 | |

| **Beispiel 9 (Emulgatorgel):** | |
|---|---|
| | Gew.-% |
| Glycerylstearatcitrat | 3,00 |
| Stearylalkohol | 1,00 |
| Ethanol | 2,00 |
| Aluminium-Stärke-Octenylsuccinat | 0,25 |
| Talkum | 0,25 |
| Xanthangummi | 0,10 |
| Retinylpalmitat | 0,20 |
| α-Liponsäure | 0,05 |
| BHT | 0,04 |
| Na₂H₂EDTA | 0,20 |
| Glycerin Parfüm, Konservierungsmittel, | 3,00 |
| Farbstoffe usw. | q.s. |
| Wasser | ad 100,00 |
| pH-Wert eingestellt auf 5.5 | |

## Patentansprüche

**1.** Kombinationen aus
(a) einem oder mehreren partiell neutralisierten Estern von Monoglyceriden und/oder Diglyceriden gesättigter Fettsäuren mit Citronensäure
(b) α-Liponsäure.

**2.** Kombinationen nach Anspruch 1, **dadurch gekennzeichnet, daß** als partiell neutralisierter Ester von Monoglyceriden und/oder Diglyceriden gesättigter Fettsäuren mit Citronensäure das Glycerylstearatcitrat gewählt wird.

**3.** Kosmetische oder dermatologische Öl-in-Wasser-Emulsion, enthaltend Kombinationen nach einem der Ansprüche 1 - 3..

**5.** Öl-in-Wasser-Emulsion nach Anspruch 4, **dadurch gekennzeichnet, daß** der oder die partiell neutralisierten Ester von Monoglyceriden und/oder Diglyceriden gesättigter Fettsäuren mit Citronensäure in kosmetischen oder topischen dermatologischen Zubereitungen in Konzentrationen von 0,1 - 20 Gew.-%, bevorzugt 0,5 - 10 Gew.-%, insbesondere 1,0 - 5,0 Gew.-% bezogen auf das Gesamtgewicht der Zubereitungen, vorliegt.
